# EUROPEAN PATENT SPECIFICATION

(11) **EP 3 268 042 B1**
(45) Date of publication and mention of the grant of the patent: **27.11.2024**
(21) Application number: 16765521.6
(22) Date of filing: 11.03.2016
(51) Int. Cl.: A61K 39/395, A61K 38/17, C07K 14/705, C07K 16/00

(54) **ANTI-TNF-ALPHA POLYPEPTIDE COMPOSITION AND USE THEREOF**
ANTI-TNF-ALPHA-POLYPEPTID-ZUSAMMENSETZUNG UND VERWENDUNG DAVON
COMPOSITION CONTENANT UN POLYPEPTIDE ANTI-TNF-ALPHA ET UTILISATION

(30) Priority: 13.03.2015 US 201562132839 P
(43) Date of publication of application: 17.01.2018
(73) Proprietor: Samsung Bioepis Co., Ltd., Incheon 406-840 (KR); Biogen MA Inc., Cambridge, MA 02142 (US)
(72) Inventor: GHIL, Jeehoon, Incheon 406-840 (KR); LEE, Yoonseok, Incheon 406-840 (KR); KIM, Songyoung, Incheon 406-840 (KR); JAQUEZ, Orlando A., 8008 Zurich (CH); GRONKE, Robert S., Boston, Massachusetts 02116 (US)
(74) Representative: Pace Napoleone, Maria
(86) International application number: PCT/US2016/022186
(87) International publication number: WO 2016/149139

(56) References cited:
- EP-A1- 3 178 847
- WO-A1-2013/006454
- WO-A1-2015/189832
- US-A1- 2005 214 291
- US-A1- 2010 266 613
- US-A1- 2014 316 114
- US-A1- 2015 037 333
- NAGAHIRA K ET AL: "Humanization of a mouse neutralizing monoclonal antibody against tumor necrosis factor-alpha (TNF-alpha)", JOURNAL OF IMMUNOLOGICAL METHODS, ELSEVIER SCIENCE PUBLISHERS B.V.,AMSTERDAM, NL, vol. 222, no. 1-2, 1 January 1999 (1999-01-01), pages 83 - 92, XP004152430, ISSN: 0022-1759, DOI: 10.1016/S0022-1759(98)00184-7
- HARDING FIONA A ET AL: "The immunogenicity of humanized and fully human antibodies: Residual immunogenicity resides in the CDR regions", 20100501, vol. 2, no. 3, 1 May 2010 (2010-05-01), pages 256 - 265, XP009137415, ISSN: 1942-0870, DOI: 10.4161/MABS.2.3.11641
- NIKO K BENDER ET AL: "Immunogenicity, efficacy and adverse events of adalimumab in RA patients", RHEUMATOLOGY INTERNATIONAL ; CLINICAL AND EXPERIMENTAL INVESTIGATIONS, SPRINGER, BERLIN, DE, vol. 27, no. 3, 28 September 2006 (2006-09-28), pages 269 - 274, XP019473129, ISSN: 1437-160X, DOI: 10.1007/S00296-006-0183-7

## Description

### BACKGROUND OF THE INVENTION

Etanercept is a recombinant human tumor necrosis factor receptor p75Fc fusion protein. Etanercept is well established and widely used in clinical practice for about 15 years, with a well characterised pharmacological, efficacy, and safety profile (Murray et al., Ann. Pharmacother., 31(11): 1335-1338 (1997); Goffet et al., J. Am. Acad. Dermatol., 49(2 Suppl): S105-S111 (2003); Fuchs et al., Clin. Trials, 1(3): 259-263 (2006)). Etanercept was originally approved for use in moderate to severe rheumatoid arthritis (RA), and the therapeutic indications for etanercept have been stepwise extended and comprise treatment of patients with polyarticular juvenile idiopathic arthritis, psoriatic arthritis, ankylosing spondylitis, psoriasis, and also paediatric psoriasis. Recently, etanercept also has been approved for use in non-radiographic axial spondyloarthritis by the EMA (Enbrel Summary of Product Characteristics. www.ema.europa.eu/docs/en_GB/document_library/EPAR_-_Product_Information/human/000262/WC500027361.pdf (retrieved on February 27, 2015)). The administration of etanercept has been found effective for treatment of some inflammatory diseases because it can reduce the levels of the active form of TNF in a subject by binding to TNF as a decoy receptor.

The administration of an anti-TNF-alpha polypeptide composition (e.g., ENBREL^{™} etanercept composition (Amgen, Inc., Thousand Oaks, California)) to a subject can produce immunogenicity against the composition (e.g., in the form of anti-drug antibodies (ADA)) in the patient. Therefore, there is a desire for an anti-TNF-alpha polypeptide composition that produces a reduced level of immunogenicity in subjects.

### BRIEF SUMMARY OF THE INVENTION

The invention provides a composition comprising an anti-tumor necrosis factor (TNF)-alpha polypeptide, wherein the composition produces a reduced level of immunogenicity when administered to a subject.

The anti-TNF-alpha polypeptide is etanercept, the composition produces a reduced level of immunogenicity in the subject as compared to an ENBREL^{™} etanercept composition with an equivalent concentration of the etanercept, and the composition is prepared by a process comprising (a) providing a mixture comprising etanercept and impurities comprising aggregates of etanercept and misfolded forms of etanercept, (b) contacting the mixture with a hydrophobic interaction chromatography (HIC) resin, such that etanercept binds to the HIC resin, and (c) eluting etanercept from the HIC resin with an elution buffer, wherein, when hydrophobic interaction high performance liquid chromatography (HI-HPLC) is performed on the composition, the composition contains a Peak 3 amount of about 12 wt.% or less.

The invention also provides the composition for use in the treatment of a disorder in which a TNF-alpha activity is detrimental in a subject wherein a therapeutically effective amount of the composition is administered to the subject.

### BRIEF DESCRIPTION OF THE DRAWINGS

Figure 1 depicts a summary of patient disposition as described in Example 3. A total of 777 patients were screened, and 181 patients were excluded mainly due to not meeting the exclusion criteria. Multiple screening failure reasons were possible. All patients randomized were included in the full analysis set and the safety set. Of the 551 patients who completed 24 weeks of treatment 481 patients were included in the per-protocol set.

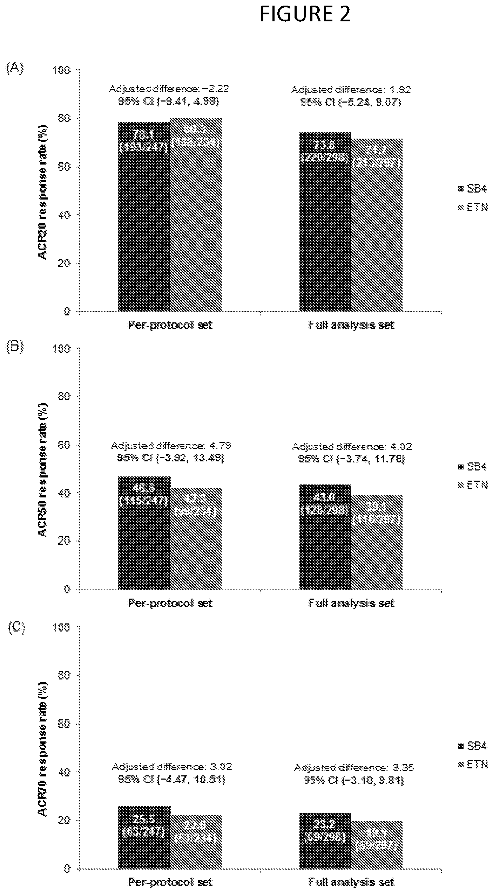

Figures 2A-2C are graphs depicting the American College of Rheumatology (ACR) response rates at Week 24 as described in Example 3. The adjusted treatment difference and its 95% confidence interval (CI) were analyzed with baseline C-reactive protein as a covariate and stratified by region. Figure 2A depicts ACR20 response rates of SB4 etanercept composition (inventive) and ETN etanercept composition (ENBREL^{™} etanercept composition) in the per-protocol set and full analysis set. Figure 2B depicts ACR50 response rates of the SB4 and ETN etanercept compositions in the per-protocol set and full analysis set. Figure 2C depicts ACR70 response rates of the SB4 and ETN etanercept compositions in the per-protocol set and full analysis set.
Figure 3 is a graph depicting estimated time-response curves of ACR20 response rate up to Week 24 in the per-protocol set as described in Example 3.
Figures 4A-4C are graphs depicting efficacy endpoints at Week 24 in the full analysis set as described in Example 3. Figure 4A depicts a change in the Disease Activity Score in 28 joints (DAS28). The LSMeans treatment difference was 0.072, and its 95% CI was -0.135 to 0.279. Figure 4B depicts the European League Against Rheumatism (EULAR) response. Figure 4C depicts the proportion of patients achieving a low disease activity score (LDAS) defined as DAS28 (ESR) ≤ 3.2 and remission defined as DAS28 (ESR) ≤ 2.6.
Figure 5 is a graph depicting the mean (standard deviation) serum through concentration (C_{trough}) profile for the SB4 and ETN etanercept compositions.
Figure 6 is a graph depicting the mean (standard deviation) serum concentration profiles at Week 8 for the SB4 and ETN etanercept compositions.

### DETAILED DESCRIPTION OF THE INVENTION

The invention provides a composition comprising an anti-tumor necrosis factor (TNF)-alpha polypeptide, wherein the composition produces a reduced level of immunogenicity when administered to a subject.

The anti-TNF-alpha polypeptide is a polypeptide that is an extracellular ligand-binding portion of a human 75 kDa (p75) TNFR fused to an Fc region of a human IgG1 (etanercept).

The composition comprising the anti-TNF-alpha polypeptide produces a reduced level of immunogenicity in a subject administered the anti-TNF-alpha polypeptide as compared to a subject administered a corresponding reference drug composition with an equivalent concentration of the anti-TNF-alpha polypeptide. The anti-TNF-alpha polypeptide is a polypeptide that is an extracellular ligand-binding portion of a human p75 TNFR fused to an Fc region of a human IgG1 (etanercept), and the composition produces a reduced level of immunogenicity in the subject as compared to an ENBREL^{™} etanercept composition (commercially available from Amgen, Inc., Thousand Oaks, California) with an equivalent concentration of the anti-TNF-alpha polypeptide.

A reduced level of immunogenicity resulting from the administration of the composition refers to a decreased immune response reaction against the composition when the composition is administered to the subject. For example, the decreased immune response reaction can be a reduced level of anti-drug-antibodies (ADA) (i.e., antibodies against the anti-TNF-alpha polypeptide in the administered composition). The anti-TNF-alpha polypeptide is a polypeptide that is an extracellular ligand-binding portion of a human p75 TNFR fused to an Fc region of a human IgG1 (etanercept), and the level of ADA is reduced by about 50% or more (e.g., about 55% or more, about 60% or more, about 65% or more, about 70% or more, about 75% or more, about 80% or more, about 85% or more, about 90% or more, about 91%, about 92%, about 93%, about 94%, about 95% or more) or by about 50-95% (e.g., about 50%, about 55%, about 60%, about 65%, about 70%, about 75%, about 80%, about 85%, about 90%, about 95%, or a range between any of these values) as compared to the ADA level in a subject administered an ENBREL^{™} etanercept composition with an equivalent concentration of the anti-TNF-alpha polypeptide.

The anti-TNF-alpha polypeptide for use in the composition (etanercept) can be produced in a known manner by recombinant DNA technology in a Chinese hamster ovary ("CHO") mammalian cell expression system. Examples in the art for mammalian expression of etanercept are described in U.S. Patent Re36755; U.S. Patent 5,605,690; U.S. Patent 7,294,481; U.S. Patent. 8,063,182; European Patent 0 417 563 B1; and European Patent 0 464 533 B1.

The anti-TNF-alpha polypeptide can be purified by any suitable means. For example, the anti-TNF-alpha polypeptide can be purified by hydroxyapatite chromatography, gel electrophoresis, dialysis, affinity chromatography, fractionation on an ion exchange column, reverse phase HPLC, chromatography on silica, chromatography on heparin, chromatography on an anion or cation exchange resin (e.g., a polyaspartic acid column), chromatofocusing, SDS-PAGE, ammonium sulfate precipitation, and/or mixed mode chromatography (see, e.g., U.S. Patent Application Publication 2014/0072560), i.e., any combination thereof.

Although not wishing to be bound by any particular theory, it is believed that the reduced level of immunogenicity that is characteristic of the composition is due at least in part to the process by which the composition is purified.

The composition is prepared by a process comprising (a) providing a mixture comprising etanercept and impurities comprising aggregates of etanercept and misfolded forms of etanercept, (b) contacting the mixture with a hydrophobic interaction chromatography (HIC) resin, such that etanercept binds to the HIC resin, and (c) eluting etanercept from the HIC resin with an elution buffer. In other words, the HIC is performed in bind-elution mode (wherein the product to be purified is bound to the HIC resin and subsequently eluted from the HIC resin).

The HIC resin can be any suitable resin. Exemplary HIC resins include, but are not limited to, Phenyl Sepharose 6 FF (High Sub), CAPTO^{™} Phenyl, CAPTO^{™} Butyl, CAPTO^{™} Phenyl ImpRes, and CAPTO^{™} Butyl ImpRes resin.

The HIC bind-elution mode provides for the removal of impurities, such as HCP, misfolded and truncated forms of the anti-TNF-alpha polypeptide, aggregates of the anti-TNF-alpha polypeptide, and other impurities (e.g., insulin, detergents, etc.). In one embodiment, the mixture of step (a), i.e., prior to contacting the mixture with a HIC resin, contains 8.0 ng/L or less (e.g., 7.5 ng/L or less, about 7 ng/L or less, about 6 ng/L or less, about 5.5 ng/L or less, about 5 ng/L or less, about 4.5 ng/L or less, or about 4 ng/L or less) of HCP.

The anti-TNF-alpha polypeptide is eluted from the HIC resin in a manner that recovers the anti-TNF-alpha polypeptide while desirably leaving a significant proportion, preferably most or all, of the aggregates (e.g., multimers such as dimers) of anti-TNF-alpha polypeptide, as well as misfolded anti-TNF-alpha polypeptides (including anti-TNF-alpha polypeptides with scrambled regions (e.g., disulfide scrambled regions) optionally bound to other proteins), bound or adhered to the HIC resin. In such a way, a significant proportion, preferably most or all, of the aggregates (e.g., multimers such as dimers) of the anti-TNF-alpha polypeptide, as well as misfolded anti-TNF-alpha polypeptides (including anti-TNF-alpha polypeptides with scrambled regions (e.g., disulfide scrambled regions) optionally bound to other proteins), in the mixture are separated from the anti-TNF-alpha polypeptide.

The anti-TNF-alpha polypeptide can be eluted from the HIC resin, typically an HIC resin column, using any suitable elution buffer. In one embodiment, the elution buffer contains about 250-400 mM (e.g., about 250 mM, about 270 mM, about 290 mM, about 300 mM, about 320 mM, about 350 mM, about 370 mM, about 400 mM, or a range between any of these values) ammonium sulfate or sodium sulfate. Alternatively, the elution buffer comprises a high concentration of salt, such as about 450-750 mM (e.g., about 450 mM, about 480 mM, about 500 mM, about 520 mM, about 550 mM, about 570 mM, about 600 mM, about 620 mM, about 650 mM, about 670 mM, about 700 mM, about 720 mM, about 750 mM, or a range between any of these values) NaCl or ammonium chloride.

Additionally, the elution buffer comprises about 10-200 mM (e.g., about 10 mM, about 20 mM, about 30 mM, about 40 mM, about 50 mM, about 60 mM, about 70 mM, about 80 mM, about 90 mM, about 100 mM, about 110 mM, about 120 mM, about 130 mM, about 140 mM, about 150 mM, about 160 mM, about 170 mM, about 180 mM, about 190 mM, about 200 mM, or a range between any of these values) of a pH modifying agent, such as sodium acetate, sodium citrate, or phosphate.

The pH of the elution buffer can be about pH 3-7 (e.g., about pH 3, about pH 3.5, about pH 4, about pH 4.5, about pH 5, about pH 5.5, about pH 6, about pH 6.5, about pH 7, or a range between any of these values. Preferably, the pH of the elution buffer is about 4.5-6.5 (e.g., about pH 4.5, about pH 4.7, about pH 5, about pH 5.2, about pH 5.5, about pH 5.7, about pH 6, about pH 6.2, about pH 6.5, or a range between any of these values).

The elution buffer can have any suitable conductivity. In one embodiment, the conductivity of the elution buffer is in a range of about 40-70 mS/cm (e.g., about 40 mS/cm, about 45 mS/cm, about 50 mS/cm, about 55 mS/cm, about 60 mS/cm, about 65 mS/cm, about 70 mS/cm, or a range between any of these values). Preferably, the conductivity of the elution buffer is less than about 65 mS/cm (e.g., about 45-60 mS/cm).

The preparation process further can comprise one or more (one, two, three, four, or five) wash steps prior to the elution step. The one or more wash steps can be performed with any suitable wash buffer. In one embodiment, the wash buffer comprises about 500-800 mM (e.g., about 500 mM, about 540 mM, about 550 mM, about 570 mM, about 600 mM, about 620 mM, about 650 mM, about 680 mM, about 700 mM, about 720 mM, about 750 mM, about 800 mM, or a range between any of these values) ammonium sulfate or sodium sulfate. Alternatively, the wash buffer comprises about 1000-1500 mM (e.g., about 1000 mM, about 1100 mM, about 1200 mM, about 1300 mM, about 1400 mM, about 1500 mM, or a range between any of these values) NaCl or ammonium chloride.

Additionally, the wash buffer comprises about 10-200 mM (e.g., about 10 mM, about 20 mM, about 30 mM, about 40 mM, about 50 mM, about 60 mM, about 70 mM, about 80 mM, about 90 mM, about 100 mM, about 110 mM, about 120 mM, about 130 mM, about 140 mM, about 150 mM, about 160 mM, about 170 mM, about 180 mM, about 190 mM, about 200 mM, or a range between any of these values) of a pH modifying agent, such as sodium acetate, sodium citrate, or phosphate.

The pH of the wash buffer can be about pH 3-7 (e.g., about pH 3, about pH 3.5, about pH 4, about pH 4.5, about pH 5, about pH 5.5, about pH 6, about pH 6.5, about pH 7, or a range between any of these values). Preferably, the pH of the wash buffer is about 4.5-6.5 (e.g., about pH 4.5, about pH 4.7, about pH 5, about pH 5.2, about pH 5.5, about pH 5.7, about pH 6, about pH 6.2, about pH 6.5, or a range between any of these values).

When the wash step is performed, the conductivity of the wash buffer desirably is greater than the conductivity of the elution buffer. Additionally, the molarity of the sum of the components of the wash buffer desirably is greater than the molarity of the sum of the components of the elution buffer (i.e., the molarity of the elution buffer is lower than the molarity of the wash buffer).

As a result of the HIC bind-elution mode, misfolded and aggregated forms of the anti-TNF-alpha polypeptide are removed from the solution comprising the anti-TNF-alpha polypeptide. As described in Example 1, the misfolded and aggregated forms of the anti-TNF-alpha polypeptide bind to the HIC resin and then are retained in the HIC resin after elution of the anti-TNF-alpha polypeptide from the HIC resin. Therefore, the resulting composition comprising the anti-TNF-alpha polypeptide has a reduced concentration of misfolded and aggregated forms of the anti-TNF-alpha polypeptide as compared to the concentration of misfolded and aggregated forms of the anti-TNF-alpha polypeptide present in the reference drug composition.

In one embodiment, the anti-TNF-alpha polypeptide is a polypeptide that is an extracellular ligand-binding portion of a human p75 TNFR fused to an Fc region of a human IgG1 (etanercept), and the concentration of high molecular weight (HMW) compounds, typically consisting of aggregates (multimer (e.g., dimer) forms of etanercept), and/or the concentration of misfolded and aggregated forms of etanercept in the resulting composition is reduced by about 50% or more (e.g., about 55% or more, about 60% or more, about 65% or more, about 70% or more, about 75% or more, about 80% or more, about 85% or more, about 90% or more, about 91%, about 92%, about 93%, about 94%, about 95% or more, or a range between any of these values) or about 50-95% (e.g., about 50%, about 55%, about 60%, about 65%, about 70%, about 75%, about 80%, about 85%, about 90%, about 95%, or a range between any of these values) as compared to the concentration of HMW compounds and/or the concentration of misfolded and aggregated forms of etanercept in the ENBREL^{™} etanercept composition.

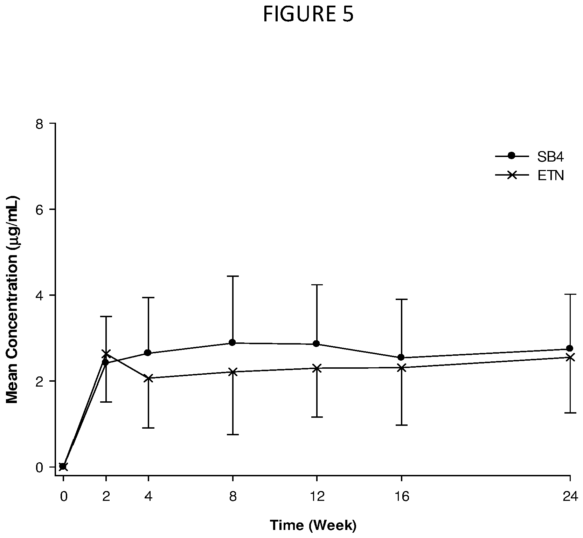

When high performance liquid chromatography (HPLC), such as hydrophobic interaction HPLC (HI-HPLC), is performed on a sample of the composition, three peaks typically are observed. As described in Example 1, Peaks 1 and 3 correspond to impurities, including truncated anti-TNF-alpha polypeptides (Peak 1), misfolded anti-TNF-alpha polypeptides (including anti-TNF-alpha polypeptides with scrambled regions (e.g., disulfide scrambled regions) optionally bound to other proteins) (Peak 3), and aggregates (e.g., multimers such as dimers) of the anti-TNF-alpha polypeptide (Peak 3), while Peak 2 corresponds to the anti-TNF-alpha polypeptide. While U.S. Patent Application Publication 2014/0072560 describes an HIC analysis of etanercept compositions produced according to a mixed mode chromatography method, the three peaks described therein (see, e.g., paragraph 0100 thereof), as well as in U.S. Patent 7,294,481 (see, e.g., column 22, lines 13-66, and Figure 5 thereof) are similar to Peaks 1, 2, and 3 described herein.

The composition contains a Peak 3 amount of about 12 wt.% or less (e.g., about 11.8 wt.% or less, about 11.5 wt.% or less, about 11.2 wt.% or less, about 11 wt.% or less, about 10.8 wt.% or less, about 10.5 wt.% or less, about 10.2 wt.% or less, about 10 wt.% or less, about 9.8 wt.% or less, about 9.5 wt.% or less, about 9.2 wt.% or less, about 9 wt.% or less, about 8.8 wt.% or less, about 8.5 wt.% or less, about 8.2 wt.% or less, about 8 wt.% or less, about 7.8 wt.% or less, about 7.5 wt.% or less, about 7.2 wt.% or less, about 7 wt.% or less, about 6.8 wt.% or less, about 6.5 wt.% or less, about 6.2 wt.% or less, about 6 wt.% or less, about 5.8 wt.% or less, about 5.5 wt.% or less, about 5.2 wt.% or less, about 5 wt.% or less, about 4.8 wt.% or less, about 4.5 wt.% or less, about 4.2 wt.% or less, about 4 wt.% or less, about 3.8 wt.% or less, about 3.5 wt.% or less, about 3.2 wt.% or less, or a range between any of these values) when HPLC is performed on a sample of the composition. Preferably, the composition contains a Peak 3 amount in a range of about 3.5-7 wt.% (e.g., about 3.5 wt.%, about 3.8 wt.%, about 4 wt.%, about 4.2 wt.%, about 4.5 wt.%, about 4.7 wt.%, about 5 wt.%, about 5.2 wt.%, about 5.3 wt.%, about 5.5 wt.%, about 5.7 wt.%, about 6 wt.%, about 6.2 wt.%, about 6.5 wt.%, about 6.7 wt.%, about 7 wt.%, or a range between any of these values). In one embodiment, the composition contains a Peak 3 amount in a range of about 3.8-7.1 wt.%.

The anti-TNF-alpha polypeptide is a polypeptide that is an extracellular ligand-binding portion of a human p75 TNFR fused to an Fc region of a human IgG1 (etanercept), and the Peak 3 amount contained in the composition is reduced by about 50% or more (e.g., about 55% or more, about 60% or more, about 65% or more, about 70% or more, about 75% or more, about 80% or more, about 85% or more, about 90% or more, about 91%, about 92%, about 93%, about 94%, about 95% or more) or by about 50-95% (e.g., about 50%, about 55%, about 60%, about 65%, about 70%, about 75%, about 80%, about 85%, about 90%, about 95%, or a range between any of these values) as compared to the Peak 3 amount contained in the ENBREL^{™} etanercept composition with an equivalent concentration of the anti-TNF-alpha polypeptide when the composition and the ENBREL^{™} etanercept composition are subjected to HPLC under the same conditions.

In another embodiment, the composition contains a Peak 2 amount of about 85 wt.% or more (e.g., about 86 wt.% or more, about 87 wt.% or more, about 88 wt.% or more, about 89 wt.% or more, about 90 wt.% or more, about 91 wt.% or more, about 92 wt.% or more, about 93 wt.% or more, about 94 wt.% or more, or a range between any of these values) when HPLC is performed on a sample of the composition.

The preparation process also can include ion (anion or cation) exchange chromatography, affinity chromatography, virus inactivation, and the like to further purify the anti-TNF-alpha polypeptide. The ion exchange chromatography can be flow-through or bind-elute mode. Any suitable ion exchange resin can be used. Exemplary ion exchange resins (i.e., chromatographic separation mediums) include, but are not limited to, HiTrap SP Sepharose FF, HiTrap CM Sepharose FF, CAPTO^{™} SP Impres, HiTrap SP HP, SOURCE 30S, HiperCel Sorbent S, CM Ceramic Hiper D, S Ceramic Hiper D, ESHMUNO S, FRACTOGEL^{™} EMD S (M), FRACTOGEL^{™} EMD S (S), FRACTOGEL^{™} EMD SE HiCap resin, FRACTOGEL^{™} TMEA HiCap, Nuvia S, UNOsphere S, Macro-Prep CM, and Macro-Prep S resin.

Thus, in one embodiment, the preparation process additionally comprises steps (a1)-(a4), which are performed prior to step (b) (contacting the mixture with the HIC resin): (a1) contacting the mixture with an ion (e.g., cation or anion) exchange resin in the presence of a load buffer, (a2) collecting a flow-through fraction containing the anti-TNF-alpha polypeptide, wherein the anti-TNF-alpha polypeptide is unbound to the ion exchange resin, (a3) applying a wash buffer and collecting a wash fraction, and (a4) pooling the flow-through fraction and the wash fraction.

Example 1 provides an exemplary description of the anti-TNF-alpha polypeptide purification process.

The composition can contain any suitable amount of the anti-TNF-alpha polypeptide. In one embodiment, the composition comprises about 10 mg/mL to about 100 mg/mL (e.g., about 20 mg/mL, about 30 mg/mL, about 40 mg/mL, about 50 mg/mL, about 60 mg/mL, about 70 mg/mL, about 80 mg/mL, about 90 mg/mL, or a range between any of these values) of anti-TNF-alpha polypeptide. Preferably, the composition comprises about 50 mg/mL of anti-TNF-alpha polypeptide.

The composition of anti-TNF-alpha polypeptide also can comprise a carrier, such as a pharmaceutically acceptable carrier or excipient that is conventional in the art and which is suitable for administration into a subject for therapeutic, diagnostic, or prophylactic purposes. The term "composition" (e.g., pharmaceutical composition) refers to a composition comprising an anti-TNF-alpha polypeptide prepared such that it is suitable for injection and/or administration into a subject in need thereof. In certain embodiments, the compositions provided herein are substantially sterile and do not contain any agents that are unduly toxic or infectious to the recipient. Further, as used herein, a solution or aqueous composition may mean a fluid (liquid) preparation that contains one or more chemical substances dissolved in a suitable solvent (e.g., water and/or other solvent, e.g., organic solvent) or mixture of mutually miscible solvents.

Such pharmaceutically acceptable carriers and excipients include, but are not limited to, additives that stabilize the polypeptide while in solution (or in dried or frozen forms), polymers, amino acids, surfactants, buffers, and combinations thereof. Suitable pharmaceutically acceptable carriers and excipients include, but are not limited to water, buffers (e.g., sodium phosphate, histidine, potassium phosphate, sodium or potassium citrate, maleic acid, ammonium acetate, tris-(hydroxymethyl)-aminomethane (tris), acetate, diethanolamine, or a combination thereof), sugars (e.g., sucrose, lactose, glycerol, xylitol, sorbitol, mannitol, maltose, inositol, trehalose, glucose, or a combination thereof), polymers (e.g., bovine serum albumin (BSA), human SA or recombinant HA, dextran, polyvinyl alcohol (PVA), hydroxypropyl methylcellulose (HPMC), polyethyleneimine, gelatin, polyvinylpyrrolidone (PVP), hydroxyethylcellulose (HEC)), non-aqueous solvents (e.g., polyethylene glycol, ethylene glycol, glycerol, dimethysulfoxide (DMSO) and dimethylformamide (DMF)), amino acids (e.g., proline, L-serine, sodium glutamic acid, alanine, glycine, lysine hydrochloride, sarcosine, gamma-aminobutyric acid), surfactants (e.g., TWEEN^{™}-20, TWEEN^{™}-80, SDS, polysorbate, and polyoxyethylene copolymer), and miscellaneous excipients (e.g., potassium phosphate, sodium acetate, ammonium sulfate, magnesium sulfate, sodium sulfate, trimethylamine N-oxide, betaine, zinc ions, copper ions, calcium ions, manganese ions, magnesium ions, CHAPS, sucrose monolaurate, 2-O-beta-mannoglycerate or a combination thereof.

The concentration of one or more carriers or excipients in the composition is about 0.0001 wt.% to about 5 wt.% (e.g., about 0.0001 wt.%, about 0.001 wt.%, about 0.01 wt.%, about 0.1 wt.%, about 0.5 wt.%, about 1 wt.%, about 1.5 wt.%, about 2 wt.%, about 2.5 wt.%, about 3 wt.%, about 3.5 wt.%, about 4 wt.%, about 4.5 wt.%, about 5 wt.%, or a range between any of these values).

In one embodiment, the composition comprises sucrose at a concentration of from about 0.5 wt.% to about 1.5 wt.% (e.g., 0.5 wt.%, 0.6 wt.%, 0.7 wt.%, 0.8 wt.%, 0.9 wt.%, 1 wt.%, 1.1 wt.%, 1.2 wt.%, 1.3 wt.%, 1.4 wt.%, 1.5 wt.%, or a range between any of these values) and preferably about 1 wt.%. The composition alternatively or additionally can comprise sodium phosphate at a concentration of about 5-15 mM (e.g., 5 mM, 6 mM, 7 mM, 8 mM, 9 mM, 10 mM, 11 mM, 12 mM, 13 mM, 14 mM, 15 mM, or a range between any of these values) and preferably about 10 mM. The composition alternatively or additionally can comprise sodium chloride at a concentration of about 100-200 mM (e.g., 100 mM, 110 mM, 120 mM, 130 mM, 140 mM, 150 mM, 160 mM, 170 mM, 180 mM, 190 mM, 200 mM, or a range between any of these values) and preferably about 140 mM.

The composition can have any suitable pH. For example, the composition can have a pH of about 5.5 to about 7.8 and preferably has a pH of about 5.8 to about 6.5 (e.g., a pH of 5.8, 5.9, 6.0, 6.1, 6.2, 6.3, 6.4, 6.5, or a range between any of these values). In one embodiment, the composition has a pH of 6.2.

In one embodiment, the composition comprises, consists essentially of, or consists of a composition comprising about 50 mg/mL of anti-TNF-alpha polypeptide, about 10 mM sodium phosphate, about 140 mM sodium chloride, and about 1 wt.% sucrose at a pH of about 6.2.

The composition is suitable for administration to a subject by any suitable mode of administration including, but not limited to, oral, aerosol, parenteral (e.g., subcutaneous, intravenous, intraarterial, intramuscular, intradermal, interperitoneal, introcerebrospinal, intrasynovial, and intrathecal), rectal, and vaginal administration. Parenteral administration can be by bolus injection or continuous infusion. Compositions for injection may be presented in unit dosage form, e.g., in ampoules or in multi-dose containers, with an added preservative.

Additionally, the composition is suitable or administration using Inject-ease GENJECT^{™} injector, injector pens such as the GENPEN^{™} injector pen, and needleless devices such as the MEDIJECTOR^{™} device and the BIOJECTOR^{™} device. In one embodiment, the composition is suitable for parental administration and is packaged in a pre-filled syringe. Preferably, the composition is suitable for subcutaneous injection.

In another embodiment, the composition is formulated as a depot preparation. Such long acting compositions may be administered by implantation (for example subcutaneously or intramuscularly) or by intramuscular injection. Thus, for example, the composition can be modified with suitable polymeric or hydrophobic materials (e.g., as an emulsion in an acceptable oil) or ion exchange resins, or as sparingly soluble derivatives, for example, as a sparingly soluble salt.

In yet another embodiment, the composition is presented in a vial, pack, or dispenser device that contains one or more unit dosage forms containing the anti-TNF-alpha polypeptide. The dispenser device can comprise a syringe having a single dose of the liquid composition ready for injection. The syringe can be accompanied by instructions for administration. The invention also provides a kit or container that comprises the composition (e.g., in aqueous form). The kit also can be accompanied by instructions for use.

The subject to be administered the composition can be any suitable subject. The subject can be a mammal, such as a mouse, rat, guinea pig, hamster, rabbit, cat, dog, pig, cow, horse, or primate (e.g., human). In one embodiment, the subject has, or is at risk for having, a disorder in which TNF-alpha activity is detrimental.

In this regard, it is disclosedthe composition for use for the treatment of a disorder in which TNF-alpha activity is detrimental in a subject wherein a therapeutically effective amount of the composition is administered to the subject.

The disorder in which TNF-alpha activity is detrimental in a subject includes, but is not limited to, rheumatoid arthritis, plaque psoriasis, psoriatic arthritis, polyarticular juvenile idiopathic arthritis (JIA), ankylosing spondylitis, Wegener's disease (granulomatosis), Crohn's disease (or inflammatory bowel disease), chronic obstructive pulmonary disease (COPD), hepatitis C, endometriosis, asthma, cachexia, psoriasis, or atopic dermatitis, or other inflammatory or autoimmune-related illness, disorder, or condition. Additional disorders that can be treated with the anti-TNF-alpha polypeptide are described in International Patent Application Publication WO 2000/062790, International Patent Application Publication WO 2001/062272, and U.S. Patent Application Publication 2011/0021380.

According to the present invention, the composition is for use in the treatment of a disorder selected from the group consisting of polyarticular JIA, psoriatic arthritis, plaque psoriasis, and ankylosing spondylitis or it is for use in the treatment of rheumatoid arthritis.

The term "treating" refers to administration or application of remedies for a disorder in a subject and includes inhibiting the disorder, arresting development of the disorder, relieving the disorder (for example, by causing regression, or restoring or repairing a lost, missing, or defective function) or stimulating an inefficient process. The term includes obtaining a desired pharmacologic and/or physiologic effect and covering any treatment of a pathological condition or disorder in a subject. The term encompasses a therapeutic effect in terms of a partial or complete cure for a disorder and/or adverse effect attributable to the disorder. Treating includes inhibiting the disorder, such as arresting its development, stopping or terminating the disorder or at least its associated symptoms, so that the subject no longer suffers from the disorder or its symptoms, such as causing regression of the disorder or its symptoms, for example, by restoring or repairing a lost, missing or defective function, or stimulating an inefficient process, or relieving, alleviating or ameliorating the disorder, or symptoms associated therewith, where ameliorating is used in a broad sense to refer to at least a reduction in the magnitude of a parameter, such as inflammation, pain and/or tumor size.

Administration of the composition to a subject can also be prophylactic. The term "preventing" encompasses complete or partial prevention of a disorder or symptom thereof, i.e., preventing the disorder from occurring or recurring in a subject who may be predisposed to the disorder but is not yet symptomatic.

The appropriate dosage, or therapeutically effective amount, of the anti-TNF-alpha polypeptide will depend on the condition to be treated, the severity of the condition, prior therapy, and the subject's clinical history and response to the anti-TNF-alpha polypeptide. The proper dose can be adjusted according to the judgment of the attending physician such that it can be administered to the subject one time or over a series of administrations. The composition can be administered as a sole therapeutic or in combination with additional therapies as needed.

In certain embodiments, the effective anti-TNF-alpha polypeptide amount per adult dose ranges from about 1-500 mg/m², or from about 1-200 mg/m², or from about 1-40 mg/m² (e.g., about 1 mg/m², about 5 mg/m², about 10 mg/m², about 15 mg/m², about 20 mg/m², about 25 mg/m², about 30 mg/m², about 35 mg/m², about 40 mg/m², or a range between any of these values) or about 5-25 mg/m². Alternatively, one dose can be administered, whose amount can range from 2-500 mg/dose, 2-100 mg/dose, or from about 10-80 mg/dose (e.g., about 10 mg/dose, about 20 mg/dose, about 30 mg/dose, about 40 mg/dose, about 50 mg/dose, about 60 mg/dose, about 70 mg/dose, about 80 mg/dose, or a range between any of these values). If the dose is to be administered more than one time per week, an exemplary dose range is the same as the foregoing described dose ranges or lower and preferably administered two or more times per week at a per dose range of 25-100 mg/dose.

In other embodiments, an acceptable dose for administration by injection contains about 80-100 mg/dose (e.g., about 80 mg/dose, about 90 mg/dose, or about 100 mg/dose). The dose can be administered at biweekly, weekly doses, or separated by several weeks (for example 2 to 8 weeks, e.g., 2 weeks, 3 weeks, 4 weeks, 5 weeks, 6 weeks, 7 weeks, or 8 weeks).

In some instances, an improvement in an subject's disorder can be obtained by a dose of up to about 100 mg of the composition one to three times per week over a period of at least three weeks, though treatment for longer periods may be necessary to induce the desired degree of improvement. For incurable chronic conditions the regimen may be continued indefinitely. For pediatric individuals (ages 2-17), a suitable regimen involves a dose of about 0.4 mg/kg to 5 mg/kg (about 0.4 mg/kg, about 0.5 mg/kg, 0.8 mg/kg, about 1 mg/kg, about 2 mg/kg, about 3 mg/kg, about 4 mg/kg, about 5 mg/kg, or a range between any of these values) of the anti-TNF-alpha polypeptide, administered one or more times per week.

The composition can be administered to the subject alone or in combination (e.g., sequential or simultaneous administration) with another active agent, such as infliximab (REMICADE^{™}), etanercept (ENBREL^{™}), adalimumab (HUMIRA^{™}), certolizumab (CIMZIA^{™}), golimumab (SIMPONI^{™}), methotrexate (MTX), or combinations thereof.

The following examples further illustrate the invention but, of course, should not be construed as in any way limiting its scope.

### EXAMPLE 1

This example describes a procedure for the preparation, particularly purification, of an SB4 etanercept composition, with a lower concentration of impurities (e.g., HCP and/or high molecular weight compounds (HMW), such as aggregates of etanercept, as well as misfolded forms of etanercept (mainly consisting of incorrect intra- or inter-disulfide bonds or aggregates) than the ENBREL^{™} etanercept composition.

The purification process comprises hydrophobic interaction chromatography (HIC), as well as possibly one or more of the following steps: ion (anion or cation) exchange chromatography, affinity chromatography, virus inactivation, and the like.

HIC is performed for removal of impurities such as HCP, Protein A leachates, misfolded and truncated forms, and aggregates. A resin such as Phenyl Sepharose 6 FF (high sub) can be used as the chromatographic separation medium and operated in bind-elute mode.

HIC can include one or more of the following steps: an equilibration step, a load step, a wash step, and an elution step.

The equilibration step equilibrates the resin to specified pH and osmolality to enable SB4 etanercept to bind. A buffer containing, for example, (a) about 0.7-1.7 M ammonium sulfate or sodium sulfate or about 1-3 M NaCl or ammonium chloride and (b) about 10-200 mM (e.g., 10-100 mM) sodium acetate, sodium citrate, or phosphate, pH 3.0-7.0, can be used.

The load step applies the SB4 etanercept mixture to the column allowing SB4 etanercept to bind while some impurities flow through.

To remove truncated forms of SB4 etanercept (HIC Peak 1), a wash step is carried out after the column is loaded. The wash step removes truncated forms of etanercept (HIC Peak 1) while retaining the target product (HIC Peak 2). The wash buffer can comprise (a) about 540-700 mM or about 1100-1500 mM NaCl or ammonium chloride and (b) about 10-200 mM sodium acetate, sodium citrate, or phosphate and have a pH of about 3-7.

The elution step elutes SB4 etanercept from the adsorbent with, for example, an elution buffer, while retaining process- and product-related impurities (HIC Peak 3) on the adsorbent. Reducing the osmolality during the elution phase allows SB4 etanercept to be collected in the effluent. Multimer forms of entanercept (HMW) and misfolded and aggregated forms of etanercept, both of which correspond to HIC Peak 3, remain bound to the HIC resin. The elution buffer can contain (a) about 250-350 mM ammonium sulfate or sodium sulfate or about 480-670 mM NaCl or ammonium chloride and (b) about 10-200 mM sodium acetate, sodium citrate, or phosphate and have a pH of about pH 3-7 and a conductivity of about 45-65 mS/cm.

Following the purification process, the SB4 etanercept composition has lower levels of impurities as compared to the ENBREL^{™} etanercept composition as shown in Table 1. In particular, the SB4 etanercept composition has a lower concentration of high molecular weight compounds (HMW) (mainly consisting of multimer forms of etanercept and aggregates) and a lower concentration of misfolded or aggregated forms of etanercept (mainly consisting of incorrect intra- or inter-disulfide bonds or aggregates) (Peak 3) as compared to the ENBREL^{™} etanercept composition when HPLC is performed.

By following the foregoing purification steps, and the SB4 etanercept composition immediately following HIC can have a Peak 3 amount in the range of about 3.5-12 wt.% (e.g., about 3.8-7.1 wt.%), while the final SB4 etanercept composition can have a Peak 3 amount in the range of about 5.2-5.3 wt.%. The results of size-exclusion (SE)-HPLC and hydrophobic interaction (HI)-HPLC analyses performed on a SB4 etanercept composition prepared using the foregoing purification process and commercially available ENBREL^{™} etanercept compositions are set forth in Table 1.

**Table 1. SB4 and ETN etanercept composition analyses**

| Assay | Units | SB4 Etanercept Composition | ENBREL^{™} Etanercept Composition | | |
|---|---|---|---|---|---|
| | | Sample (N=3) | Sample 1 (N=3) | Sample 2 (N=3) | Sample 3 (N=3) |
| SE-HPLC | wt.% HMW | 1.8-2.2 | 2.7-3.1 | 2.0-3.2 | 2.5-3.9 |
| HI-HPLC | wt.% Peak 3 | 5.2-5.3 | 13.5-13.6 | 15.1-17.5 | 14.2-14.8 |

### EXAMPLE 2

This example provides materials and methods for the study described in Example 3.

### Patients

Patients aged 18-75 years who have been diagnosed with rheumatoid arthritis (RA) according to the revised 1987 American College of Rheumatology (ACR) criteria for ≥ 6 months and ≤ 15 years prior to screening were eligible for the study. Patients had to have active disease defined as: ≥ 6 swollen and ≥ 6 tender joints and either erythrocyte sedimentation rate (ESR) ≥ 28 mm/h or serum C-reactive protein (CRP) ≥ 1.0 mg/dL despite MTX treatment for ≥ 6 months (stable dose of 10-25 mg/week for ≥ 4 weeks prior to screening). Non-steroidal anti-inflammatory drugs and oral glucocorticoids (equivalent to ≤ 10 mg prednisolone) were permitted if received at a stable dose for ≥ 4 weeks prior to randomisation.

Major exclusion criteria consisted of previous treatment with any biological agents, history of lymphoproliferative disease, congestive heart failure (New York Heart Association Class III/IV), or demyelinating disorders, diagnosis of active tuberculosis (TB), and pregnancy or breastfeeding at screening.

### Study Design

This Phase III, randomised, double-blind, parallel group study was conducted at 73 centers across 10 countries in Europe, Latin America, and Asia. Patients were randomized in a 1:1 ratio to receive 50 mg of either SB4 etanercept composition or ENBREL^{™} etanercept composition (ETN) once-weekly for up to 52 weeks via subcutaneous selfadministration. All patients had to take methotrexate (MTX) (10-25 mg/week) and folic acid (5-10 mg/week) during the study. This report represents efficacy data up to 24 weeks of treatment and safety data up to the 24-week interim report data cut off point (July 21, 2014).

### Study Endpoints

The primary endpoint was to demonstrate equivalence between SB4 and ETN etanercept compositions at Week 24, in terms of ACR20 response rate. Other efficacy endpoints were the ACR50 and ACR70 responses, change in the disease activity score based on a 28 joint count (DAS28), and the EULAR response. Safety endpoints included incidence of adverse events (AEs) and serious adverse events (SAEs).

Blood samples for PK analyses were collected from a subset of patients (PK population) at designated study sites. Key PK endpoints included serum trough concentration (C_{trough}) and area under the concentration-time curve during the dosing interval (AUC_{τ}) at steady state. Serum concentrations were determined using a validated enzyme-linked immunosorbent assay (ELISA) and PK parameters were calculated by non-compartmental analyses (WinNonlin version 5.2 or higher, Pharsight, Mountain View, CA).

Immunogenicity endpoints were incidence of anti-drug antibodies (ADAs) and neutralizing antibodies (NAbs). A single assay approach with SB4 etanercept tag was used to assess immunogenicity. ADAs were measured using validated electrochemiluminesence (ECL) immunoassays (MesoScale Discovery [MSD] platform) and NAbs were measured using a competitive ligand-binding assay.

### Statistical Analyses

Sample size was determined using the historical data for the equivalence test. The expected ACR20 response rate at Week 24 for both SB4 and ETN etanercept compositions was expected to be 60% from the previous ETN etanercept composition pivotal studies (Weinblatt et al., N. Engl. J. Med., 340(4): 253-259 (1999); Combe et al., Ann. Rheum. Dis., 65(10): 1357-1362 (2006); and Keystone et al., Arthritis Rheum., 50(2): 353-363 (2004)). Based on the expected response rate, the equivalence margin of [-15%, 15%] at Week 24 for ACR20 response rate was calculated in line with the FDA Guidance for Industry Non-Inferiority Clinical Trials and CHMP Guideline on the Choice of the Non-inferiority Margin and was also accepted by the regulatory agencies (U.S. Department of Health and Human Services, Food and Drug Administration, Guidance for Industry: Non-inferiority clinical trials, www.fda.gov/downloads/Drugs/Guidances/UCM202140.pdf (February 27 2015); and Committee for Medicinal Products for Human Use (CHMP), Guideline on the choice of the non-inferiority margin (EMEA/CPMP/EWP/2158/99) (July 27, 2005), www.ema.europa.eu/ docs/en_GB/document_library/Scientific_guideline/2009/09/WC500003636.pdf (February 27, 2015).

Given a two-sided α level of 0.05 and 80% power, the two-sided 15% equivalence margin required 438 patients for the per-protocol set (PPS). Assuming 20% loss of patients from the PPS, the study required a minimum of 548 randomized patients.

The primary efficacy analysis for ACR20 response at Week 24 was performed on the PPS in which patients completed Week 24 visit, received 80-120% of both the expected number of IP injections and the expected sum of MTX doses, and did not have any major protocol deviations affecting the efficacy assessment. To declare the equivalence between the two treatment groups, the 95% confidence interval (CI) of the adjusted treatment difference had to be entirely contained within the equivalence margin of [-15%, 15%]. The 95% CI of the difference of ACR20 response rates was estimated non-parametrically using the Mantel-Haenszel weights for region while adjusting for the baseline CRP. As a sensitivity analysis, the same analysis was repeated for the full analysis set (FAS) with missing data at Week 24 considered as non-responses to explore the robustness of the results. Similar analyses were performed for ACR50 and ACR70 responses at Week 24.

In addition, the exponential time-response model for ACR20 response rate was used to investigate the treatment difference during the time course of the study up to Week 24 (Weinblatt et al., N. Engl. J. Med., 340(4): 253-259 (1999)). Each exponential time-response model for ACR20 response rate was fitted by the non-linear mixed model for SB4 and ETN etanercept compositions separately, and the difference of those models and the 95% CI were calculated using the 2-norm criterion which measured the squared differences across all timepoints. To declare the equivalence between two treatment groups using the time-response models, the upper limit of 95% CI of the difference between the models had to be less than the pre-specified equivalence margin, which was calculated from the historical data using the 2-norm criterion (Reeve et al., Therapeutic Innovation & Regulatory Science, 47(6): 641-650 (2013)).

Safety and immunogenicity endpoints were analysed descriptively on the safety set which included all patients who received at least one dose of study drug. PK endpoints were summarised descriptively on the PK population which included patients in the safety set who had at least one PK sample collected.

The analyses were performed using SAS version 9.2 software (SAS Institute, Cary, North Carolina, USA).

### EXAMPLE 3

This example describes a randomized, double-blind, parallel group, multicenter study.

The efficacy, safety, and immunogenicity of SB4 etanercept composition prepared using the process described in Example 1 were compared to those of ENBREL^{™} etanercept product (ETN) in patients with moderate to severe RA despite MTX treatment.

### Patient disposition and baseline characteristics

Patient screening began in June 2013, and the 24-week evaluation of the last patient occurred in April 2014. Overall, 777 patients were screened, of whom 596 patients were randomized. A total of 551 patients completed 24 weeks of treatment and 481 (80.7%) patients were included in the PPS (70 patients were excluded from the PPS due to protocol deviations) (see Table 2).

**Table 2. Protocol deviations**

| Protocol deviations | | SB4 50 mg | ETN 50 mg | Total |
|---|---|---|---|---|
| | | N=299 | N=297 | N=596 |
| | | n (%) | n (%) | n (%) |
| With at least one major protocol deviation | | 73 (24.4) | 72 (24.2) | 145 (24.3) |
| Excluded from Per-protocol set | | 40 (13.4) | 35 (11.8) | 75 (12.6) |
| | Concomitant medication criteria | 9 (3.0) | 14 (4.7) | 23 (3.9) |
| | Eligibility and entry criteria | 7 (2.3) | 5 (1.7) | 12 (2.0) |
| | Investigational product compliance | 9 (3.0) | 2 (0.7) | 11 (1.8) |
| | Study procedures criteria | 16 (5.4) | 16 (5.4) | 32 (5.4) |

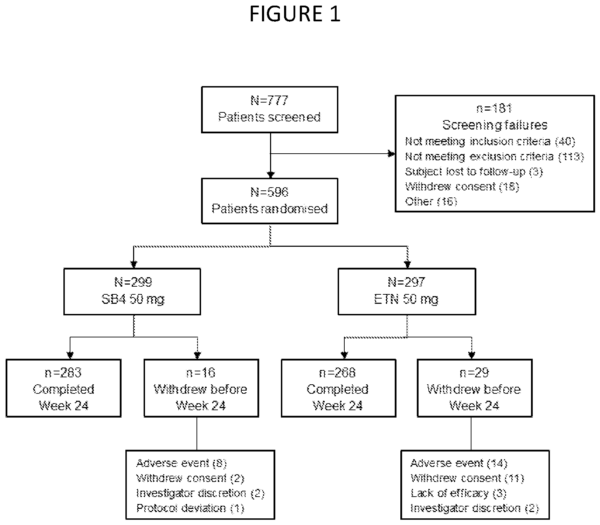

Patients withdrew before Week 24 mainly due to AEs (3.7%) and withdrawal of consent (2.7%) (see Figure 1). The demographic and baseline disease characteristics were comparable between treatment groups (see Table 3).

**Table 3. Baseline demographics and disease characteristics**

| | | SB4 50 mg | ETN 50 mg | Total |
|---|---|---|---|---|
| | | N=299 | N=297 | N=596 |
| Age (years), mean (SD) | | 52.1 (11.72) | 51.6(11.63) | 51.8 (11.67) |
| Age group, n (%) | | | | |
| | < 65 years | 253 (84.6) | 262 (88.2) | 515 (86.4) |
| | ≥ 65 years | 46 (15.4) | 35 (11.8) | 81 (13.6) |
| Gender n (%) | | | | |
| | Male | 50 (16.7) | 44 (14.8) | 94 (15.8) |
| | Female | 249 (83.3) | 253 (85.2) | 502 (84.2) |
| Race, n (%) | | | | |
| | White | 279 (93.3) | 273 (91.9) | 552 (92.6) |
| | American Indian or Alaskan Native | 5 (1.7) | 7 (2.4) | 12 (2.0) |
| | Asian | 11 (3.7) | 13 (4.4) | 24 (4.0) |
| | Other | 4 (1.3) | 4 (1.3) | 8 (1.3) |
| Weight (kg) , mean (SD) | | 72.5 (15.93) | 71.0 (14.63) | 71.8 (15.30) |
| Height (cm), mean (SD) | | 164.4 (8.78) | 164.4 (8.55) | 164.4 (8.66) |
| BMI (kg/m²), mean (SD) | | 26.8 (5.51) | 26.3 (5.30) | 26.6 (5.41) |
| Disease duration (years), mean (SD) | | 6.0 (4.20) | 6.2 (4.41) | 6.1 (4.30) |
| Duration of MTX use (months), mean (SD) | | 48.2 (39.87) | 47.1 (40.77) | 47.7 (40.29) |
| MTX dose (mg/week), mean (SD) | | 15.6 (4.52) | 15.5 (4.60) | 15.5 (4.56) |
| Swollen joint count (0-66), mean (SD) | | 15.4 (7.48) | 15.0 (7.30) | 15.2 (7.39) |
| Tender joint count (0-68), mean (SD) | | 23.5 (11.90) | 23.6 (12.64) | 23.5 (12.26) |
| HAQ-DI (0-3), mean (SD) | | 1.49 (0.553) | 1.50 (0.560) | 1.50 (0.556) |
| Physician global assessment VAS (0-100), mean (SD) | | 62.2 (15.09) | 63.2 (14.76) | 62.7 (14.92) |
| Subject global assessment VAS (0-100), mean (SD) | | 61.7 (18.97) | 63.0 (17.70) | 62.4 (18.35) |
| Subject pain assessment VAS (0-100), mean (SD) | | 61.8 (20.22) | 62.3 (19.22) | 62.1 (19.71) |
| DAS28 (ESR), mean (SD) | | 6.5 (0.91) | 6.5 (0.88) | 6.5 (0.89) |
| C-reactive protein (mg/dL), mean (SD) | | 1.5 (2.00) | 1.3 (1.60) | 1.4 (1.81) |
| Erythrocyte sedimentation rate (mm/h), mean (SD) | | 46.5 (22.10) | 46.4 (22.62) | 46.5 (22.34) |
| Rheumatoid factor positive, n (%) | | 237 (79.3) | 231 (77.8) | 468 (78.5) |

BMI, body mass index; DAS28, disease activity score based on 28 joint count; ETN, reference product etanercept; HAQ-DI, health assessment questionnaire-disability index; MTX, methotrexate; SD, standard deviation; VAS, visual analogue scale.

### Efficacy

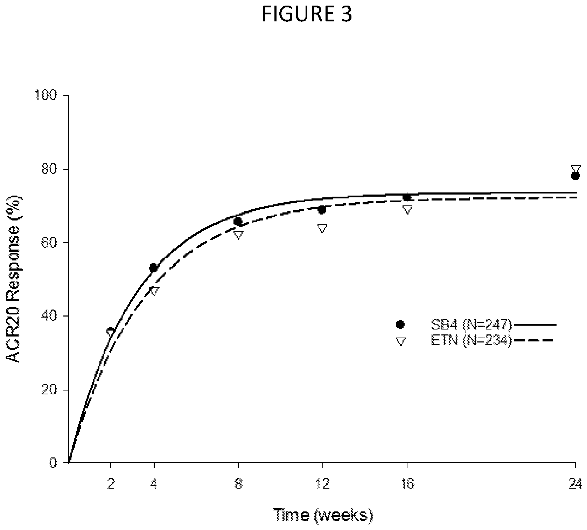

The ACR20 response rate at Week 24 in the PPS was 78.1% for SB4 etanercept composition and 80.3% for ETN etanercept composition. The 95% CI of the adjusted difference (SB4 - ETN) in ACR20 response rate was within the pre-defined equivalence margin of [-15%, 15%] in both the PPS (95% CI: -9.41%, 4.98%) and FAS (95% CI: -5.24%, 9.07%), indicating therapeutic equivalence between SB4 and ETN etanercept compositions (see Figure 2). The time-response models of SB4 and ETN etanercept compositions up to Week 24 in the PPS were estimated to be equivalent since the treatment difference in terms of the 2-norm difference was 12.7 and the 95% CI was (-4.6, 30.0), where the upper limit 30.0 was less than the pre-specified equivalence margin of 83.28 (see Figure 3).

The ACR50 and ACR70 response rates at Week 24 in the PPS and FAS were equivalent between SB4 and ETN etanercept compositions. The ACR50 response rate was 46.6% vs. 42.3% and the ACR70 response rate was 25.5% vs. 22.6% in the PPS for SB4 and ETN etanercept compositions, respectively (see Figure 2).

Subgroup analyses on the ACR response rates in PPS showed comparable results regardless of ADA status. The proportion of patients who achieved ACR20 response rate among patients who tested negative for antibodies against etanercept was 78.0% for the SB4 etanercept composition and 81.5% for the ETN etanercept composition (95% CI: -11.12%, 3.99%) (see Table 4).

**Table 4. Analysis of ACR20 response rate at Week 24 by overall 24-week ADA status**

| 24-week ADA status | Treatment | n/n' | (%) | Adjusted Difference (SE) | 95% CI | p-value |
|---|---|---|---|---|---|---|
| Positive | SB4 50 mg (N=2) | 2/2 | (100.0) | 22.14% | (-54.79%, | |
| | ETN 50 mg (N=29) | 21/29 | (72.4) | (37.095%) | 99.07%) | |
| Negative | SB4 50 mg (N=245) | 191/245 | (78.0) | -3.57% | (-11.12%, | |
| | ETN 50 mg (N=205) | 167/205 | (81.5) | (3.846%) | 3.99%) | |
| | | | | | | 0.292 |

| | | | | | | |
|---|---|---|---|---|---|---|
| ADA status was defined as positive if patient had a positive test result at least once up to Week 24. The adjusted difference and its 95% CI were estimated by analysis of covariance model with treatment and region as factors and baseline C-reactive protein value as covariate. ADA, anti-drug antibody; CI, confidence interval; SE, standard error. | | | | | | |

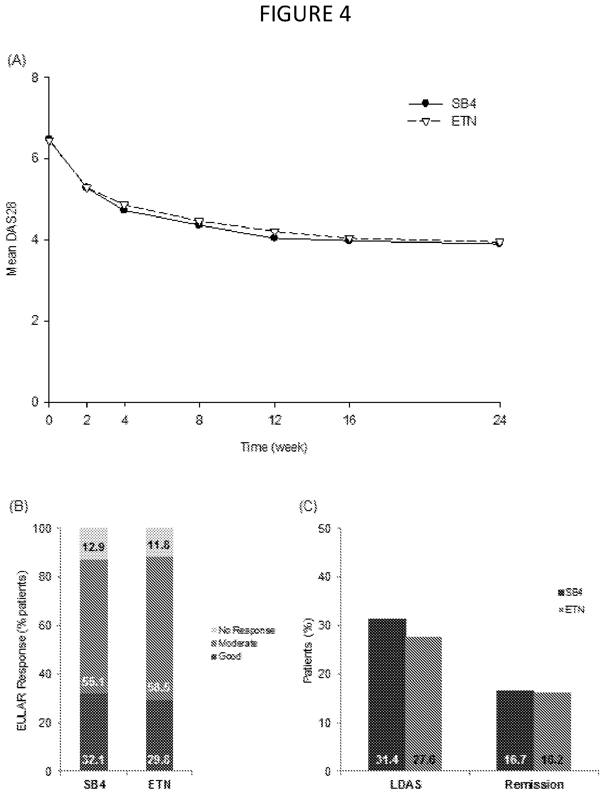

The mean improvement in DAS28 (ESR) score from baseline was 2.6 and 2.5 at Week 24 for the SB4 and ETN etanercept compositions, respectively (95% CI: -0.14, 0.28) (see Figure 4A). The proportion of patients achieving good or moderate EULAR response (see Figure 4B), low disease activity score, or remission (see Figure 4C) at Week 24 according to DAS28 were similar between SB4 and ETN etanercept compositions. The ACR-N at Week 24 was 45.0% for the SB4 etanercept composition and 43.7% for the ETN etanercept composition. The area under the curve (AUC) of ACR-N up to Week 24 (5822.2 vs. 5525.0) and change in DAS28 from baseline up to Week 24 (358.3 vs. 343.5) were comparable between SB4 and ETN etanercept compositions.

The primary endpoint at Week 24 was met: the 95% CI of the adjusted treatment difference between the SB4 and ETN etanercept compositions in ACR20 response rate was within the pre-defined equivalence margin of [-15%, 15%]. The ACR20 responses observed in this study (73.8% for the SB4 etanercept composition and 71.7% for the ETN etanercept composition in FAS) were within the range of ACR20 response rates reported in pivotal studies with the ETN etanercept composition (74% in Combe et al., Ann. Rheum. Dis., 65(10): 1357-1362 (2006), 72% in Genovese et al., Arthritis Rheum., 46(6): 1443-1450 (2002), 71% in Weinblatt et al., N. Engl. J. Med., 340(4): 253-259 (1999), 59% in Moreland et al., Ann. Intern. Med., 130(6): 478-486 (1999), and 49% in Keystone et al., Arthritis Rheum., 50(2): 353-363 (2004)).

As the primary efficacy assessment (ACR20 response at Week 24) was evaluated at a time point in the therapeutic plateau, various efficacy endpoints and statistical methods were applied to detect any non-equivalence in efficacy and to support the robustness of the primary efficacy analysis. The ACR20 response rate, ACR-N, and DAS28 were measured at several different time points early in the treatment period. The time-response curves for the SB4 and ETN etanercept compositions up to Week 24 showing the ACR20 response over time were estimated to be equivalent and the AUC of ACR-N up to Week 24 and AUC of the change in DAS28 (ESR) from baseline up to Week 24 were comparable between the SB4 and ETN etanercept compositions indicating that the efficacy of the SB4 etanercept composition over time was similar to the efficacy of the ETN etanercept composition over time.

### Safety

Overall, 165 (55.2%) patients treated with the SB4 etanercept composition and 173 (58.2%) patients treated with the ETN etanercept composition reported at least one TEAE. Frequently occurring TEAEs by preferred term are shown in Table 5 and the most frequently reported TEAE were upper respiratory tract infection (7.0%) and alanine aminotransferase increased (5.0%) for the SB4 etanercept composition and injection site erythema (11.1%), upper respiratory tract infection (5.1%), and nasopharyngitis (5.1%) for the ETN etanercept composition. Most of the TEAEs were mild to moderate in severity and TEAEs considered related to the study drug were reported in 83 (27.8%) and 106 (35.7%) patients for the SB4 and ETN etanercept compositions, respectively. Serious TEAEs were reported in 13 patients for each of the SB4 and ETN etanercept compositions and 34 patients discontinued treatment due to TEAE (15 [5.0%] patients vs. 19 [6.4%] patients).

**Table 5. Treatment-emergent adverse events reported in ≥ 2% patients by preferred term, n (%)**

| Preferred term | SB4 50 mg | ETN 50 mg |
|---|---|---|
| | N=299 | N=297 |
| Upper respiratory tract infection | 21 (7.0) | 15 (5.1) |
| Alanine aminotransferase increased | 15 (5.0) | 14(4.7) |
| Nasopharyngitis | 14 (4.7) | 15 (5.1) |
| Headache | 13 (4.3) | 8 (2.7) |
| Hypertension | 10 (3.3) | 10 (3.4) |
| Aspartate aminotransferase increased | 7 (2.3) | 8 (2.7) |
| Viral infection | 7 (2.3) | 5 (1.7) |
| Injection site erythema | 6 (2.0) | 33 (11.1) |
| Rheumatoid arthritis | 6 (2.0) | 9 (3.0) |
| Bronchitis | 6 (2.0) | 6 (2.0) |
| Diarrhea | 5 (1.7) | 7 (2.4) |
| Pharyngitis | 4 (1.3) | 8 (2.7) |
| Urinary tract infection | 4 (1.3) | 7 (2.4) |
| Lymphocyte count decreased | 4 (1.3) | 6 (2.0) |
| Cough | 3 (1.0) | 10 (3.4) |
| Erythema | 2 (0.7) | 10 (3.4) |
| Dizziness | 2 (0.7) | 7 (2.4) |
| Injection site rash | 2 (0.7) | 6 (2.0) |
| Injection site reaction | 1 (0.3) | 7 (2.4) |

A total of 27 patients (12 patients for the SB4 etanercept composition and 15 patients for the ETN etanercept composition) were diagnosed at screening with latent TB but entered the study while receiving treatment for latent TB. None of these patients or any other patients developed active TB during the study. Other serious infections were reported in 1 (0.3%) patient treated with the SB4 etanercept composition and 4 (1.3%) patients treated with the ETN etanercept composition. Malignancies were reported in 3 (1.0%) patients treated with the SB4 etanercept composition (basal cell carcinoma, breast cancer, and lung cancer metastatic) and in 1 (0.3%) patient treated with the ETN etanercept composition (invasive ductal breast carcinoma).

Injection site reactions, counted by the high-level group term of administration site reaction, occurred in fewer patients treated with the SB4 etanercept composition compared to the ETN etanercept composition. There were 22 injection site reaction reported in 11 (3.7%) patients vs. 156 injection site reactions reported in 51 (17.2%) patients treated with the SB4 and ETN etanercept compositions, respectively. Most of the injection site reactions occurred early (between Week 2 and Week 8) and were mild in severity and none led to study drug discontinuation. The proportion of injection site reactions for the SB4 and ETN etanercept compositions were 3.7% vs. 17.1% in ADA positive patients and 0.0% vs. 17.9% in ADA negative patients, respectively (see Table 6).

**Table 6. Injection site reaction by overall 24-week ADA status**

| | | SB4 (N=299) | | | ETN (N=297) | | |
|---|---|---|---|---|---|---|---|
| Overall 24-week ADA status | | n/n' | (%) | E | n/n' | (%) | E |
| | Positive | 0/2 | (0.0) | 0 | 7/39 | (17.9) | 23 |
| | Negative | 11/297 | (3.7) | 22 | 44/258 | (17.1) | 133 |

| | | | | | | | |
|---|---|---|---|---|---|---|---|
| ADA, anti-drug antibody; E, events; n': number of patients with available overall 24-week ADA assessment results; percentages are based on n'. n: number of patients who have injection site reactions counted by the high-level group term (HLGT) of administration site reaction. Overall 24-week ADA result was defined as positive for patients with at least one ADA positive result up to Week 24 after Week 0. | | | | | | | |

Overall, the safety profile of the SB4 etanercept composition was comparable with that of the ETN etanercept composition and was similar to those observed in the pivotal trials with the ETN etanercept composition. There were no cases of active tuberculosis, and only 1 patient treated with the SB4 etanercept composition and 4 patients treated with the ETN etanercept composition reported serious infection which is lower than 6.3% shown in ETN etanercept composition product information (Enbrel Summary of Product Characteristics, www.ema.europa.eu/docs/en_GB/document_library/EPAR_-_Product_Information/human/ 000262/WC500027361.pdf (retrieved on February 27, 2015). Malignancies were reported in 3 (1.0%) patients treated with the SB4 etanercept composition and 1 (0.3%) patients treated with the ETN etanercept composition. The incidence of malignancy observed in this study is similar to the previously conducted studies (Weinblatt et al., N. Engl. J. Med., 340(4): 253-259 (1999); Lopez-Olivo et al., Jama, 308(9): 898-908 (2012); and Combe et al., Ann. Rheum. Dis., 68(7): 1146-1152 (2009)).

Interestingly, injection site reactions were reported in fewer patients treated with the SB4 etanercept composition compared to the ETN etanercept composition (3.7% vs. 17.2%). The overall incidence of injection site reaction occurred in this study (10.2%) is in line with previously conducted studies, and most injection site reactions occurred in the first month which is in accordance with the reference product label (Enbrel Summary of Product Characteristics. www.ema.europa.eu/docs/en_GB/document_library/EPAR_-_Product_ Information/human/000262/WC500027361.pdf (retrieved on February 27, 2015). There appears to be no correlation between injection site reaction and ADA development, which is consistent with previously conducted studies (Dore et al., Clin. Exp. Rheumatol., 25(1): 40-46 (2007)).

### Pharmacokinetics

PK analyses were performed on 78 patients (41 patients treated with the SB4 etanercept composition and 38 patients treated with the ETN etanercept composition).

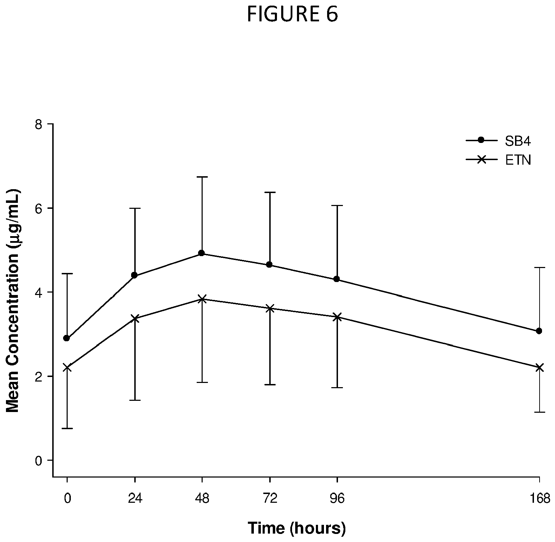

C_{trough} were comparable at each time point for the SB4 etanercept composition (ranging from 2.419 to 2.886 µg/mL in Weeks 2 to 24) and the ETN etanercept composition (ranging from 2.066 to 2.635 µg/mL in Weeks 2 to 24) (see Figure 5). The AUC_{τ} at Week 8 was 676.4 vs. 520.9 µg/mL and the inter-subject variability (CV%) was 37.7% vs. 50.1% for the patients treated with the SB4 and ETN etanercept compositions, respectively (see Figure 6).

In this study C_{trough} and steady state PK were investigated in a subset of population to provide supporting evidence to the Phase I comparative PK study in healthy subjects that demonstrated similar PK behavior. In the Phase III study, the C_{trough} values were comparable between the SB4 and ETN etanercept compositions at each time point and AUC_{τ} at steady state was relatively higher for the SB4 etanercept composition compared to the ETN etanercept composition; however the numerical difference is likely due to an inherent high inter-subject variability (37.7% vs. 50.1%).

### Immunogenicity

The incidence of ADA was significantly lower in patients treated with the SB4 etanercept composition compared to the ETN etanercept composition. Two (0.7%) patients treated with the SB4 etanercept composition and 39 (13.1%) patients treated with the ETN etanercept composition tested positive at least once up to Week 24 (p < 0.001), and only one sample from the ETN group had neutralizing capacity. The ADAs appeared early (between Week 2 and Week 8) and most of the ADAs disappeared after Week 12.

The characteristics of antibodies detected in this study were generally transient and non-neutralizing which is in accordance with those established with the ETN etanercept composition in previous studies of Product Characteristics: www.ema.europa.eu/docs/ en_GB/document_library/EPAR_-_Product_Information/human/000262/WC500027361.pdf (retrieved on February 27, 2015); and Dore et al., Clin. Exp. Rheumatol., 25(1): 40-46 (2011)). Since SB4 etanercept tagged single assay approach was used to detect immunogenicity, the assay method does not seem to have caused the lower incidence of ADA observed in patients treated with the SB4 etanercept composition compared to the ETN etanercept composition (0.6% vs. 13.1%). The lower immunogenicity of the SB4 etanercept composition does not preclude classification as biosimilar because clinical efficacy of the SB4 and ETN etanercept compositions were equivalent in patients with ADA negative results and no apparent correlation between ADA and clinical response or safety was observed (European Medicines Agency, Guideline on similar biological medicinal products containing biotechnology-derived proteins as active substance: non-clinical and clinical issues (EMEA/CHMP/BMWP/42832/2005) (February 22, 2006), www.ema.europa.eu/docs/en_GB/ document_library/Scientific_guideline/2009/09/WC500003920.pdf (retrieved on February 27, 2015); and Dore et al., Clin. Exp. Rheumatol., 25(1): 40-46 (2011)).

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. A composition comprising an anti-tumor necrosis factor (TNF)-alpha polypeptide,
wherein the anti-TNF-alpha polypeptide is etanercept,
wherein the composition is prepared by a process comprising:
(a) providing a mixture comprising etanercept and impurities comprising aggregates of etanercept and misfolded forms of etanercept,
(b) contacting the mixture with a hydrophobic interaction chromatography (HIC) resin, such that etanercept binds to the HIC resin, and
(c) eluting etanercept from the HIC resin with an elution buffer, thereby obtaining the composition comprising etanercept
wherein said composition produces a reduced level of immunogenicity in the subject as compared to an ENBREL^{™} etanercept composition with an equivalent concentration of the etanercept, and
wherein, when hydrophobic interaction high performance liquid chromatography (HI-HPLC) is performed on the composition, the composition contains a Peak 3 amount of about 12 wt.% or less.

2. The composition according to claim 1 wherein the elution buffer comprises (a) about 250-400 mM ammonium sulfate or sodium sulfate or about 450-750 mM NaCl or ammonium chloride and (b) about 10-200 mM sodium acetate, sodium citrate, or phosphate.

3. The composition according to claim 1 or 2 wherein the elution buffer has a pH of 3-7.

4. The composition of claim 1-3, wherein the process further comprises step (b1) prior to step (c): (b1) washing impurities from the HIC resin with a wash buffer.

5. The composition of claim 4, wherein the wash buffer comprises (a) about 500-800 mM ammonium sulfate or sodium sulfate or about 1000-1500 mM NaCl or ammonium chloride and (b) about 10-200 mM of sodium acetate, sodium citrate or phosphate;

6. The composition of claim 4 or 5, wherein the wash buffer has a pH of 3-7.

7. The composition of claim 4-6, wherein the conductivity of the wash buffer is greater than the conductivity of the elution buffer, or the molarity of the elution buffer is lower than the molarity of the wash buffer.

8. The composition of any one of claims 1-7, wherein the reduced level of immunogenicity is a reduced level of anti-drug antibodies (ADA) which is reduced by 50%, 60%, 70%, 80%, 90% or more relative to a subject administered an ENBREL^{™} etanercept composition with an equivalent concentration of etanercept.

9. The composition of any one of claim 1-8, wherein the composition is packaged in a pre-filled syringe.

10. The composition of any one of claims 1-9, wherein the composition is suitable for subcutaneous injection.

11. The composition of any one of claims 1-10, wherein the mixture of step (a) contains 8.0 ng/L or less of host cell proteins (HCP).

12. The composition of any one of claim 1-11, wherein, when hydrophobic interaction high performance liquid chromatography (HI-HPLC) is performed on the composition, the composition contains a Peak 3 amount of about 7 wt.% or less.

13. The composition of claim 12, wherein the composition contains a Peak 3 amount in a range of about 3.5-7 wt %.

14. The composition of claim 12 or 13, wherein the Peak 3 amount is reduced by 50%, 60%, 70% or more relative to the Peak 3 amount contained in the ENBREL^{™} etanercept composition with an equivalent concentration of etanercept and subjected to HPLC under the same conditions.

15. The composition of any one of claim 1-14, wherein the process additionally comprises one or more of the following: ion exchange chromatography, affinity chromatography, size exclusion chromatography, and virus inactivation.

16. The composition of any one of claims 1-15, comprising 10 mg/mL to 100 mg/mL of etanercept or 50 mg/mL of etanercept.

17. The composition of any one of claims 1-16, wherein the concentration of misfolded and aggregated forms of etanercept in the compositions is reduced by 50%, 60%, 70%, 80%, 90% or more relative to the concentration of misfolded and aggregated forms of etanercept in the ENBREL^{™} etanercept composition.

18. The composition of any one of claims 1-17 wherein the concentration of high molecular weight compounds in the composition is reduced 50%, 60%, 70%, 80%, 90% or more relative to the concentration of high molecular weight compounds in the ENBREL^{™} etanercept composition.

19. The composition of any one of claim 1-18 for use in the treatment of a disorder selected from the group consisting of polyarticular juvenile idiopathic arthritis (JIA), psoriatic arthritis, plaque psoriasis, and ankylosing spondylitis, or for use in the treatment of rheumatoid arthritis.

20. The composition for use according to claim 19, wherein the composition is administered subcutaneously.

21. The composition for use according to claim 19, wherein in the subject the level of ADA is reduced by 50%, 60%, 70%, 80%, 90% or more relative to a subject administered an ENBREL^{™} etanercept composition with an equivalent concentration of etanercept.

22. A method to obtain a composition comprising an anti-tumor necrosis factor (TNF)-alpha polypeptide wherein the anti-TNF-alpha polypeptide is etanercept, comprising:
(a) providing a mixture comprising etanercept and impurities comprising aggregates and misfolded etanercept,
(b) contacting the mixture with a hydrophobic interaction chromatography (HIC) resin, such that etanercept binds to the HIC resin, and
(c) eluting the etanercept from the HIC resin with an elution buffer,
(d) obtaining the composition comprising etanercept
wherein said composition produces a reduced level of immunogenicity when administered to a subject as compared to an ENBREL^{™} etanercept composition with an equivalent concentration of etanercept.

## Patentansprüche

1. Zusammensetzung umfassend ein Anti-Tumor-Nekrose-Faktor- (TNF) alpha-Polypeptid,
wobei das Anti-TNF-alpha-Polypeptid Etanercept ist,
wobei die Zusammensetzung durch ein Verfahren hergestellt wird, das Folgendes umfasst:
(a) Bereitstellen einer Mischung umfassend Etanercept und Verunreinigungen umfassend Anhäufungen von Etanercept und fehlgefalteten Formen von Etanercept,
(b) Kontaktieren der Mischung mit einem hydrophoben Wechselwirkungschromatographie- (HIC) Harz, derart, dass das Etanercept sich an das HIC-Harz bindet, und
(c) Eluieren von Etanercept aus dem HIC-Harz mit einem Elutionspuffer, wodurch die Etanercept umfassende Zusammensetzung erhalten wird,
wobei die Zusammensetzung ein reduziertes Niveau von Immunogenität in dem Subjekt im Vergleich mit einer ENBREL^{WZ}-Etanercept-Zusammensetzung mit einer äquivalenten Konzentration des Etanercepts erzeugt und
wobei, wenn hydrophobe Wechselwirkungshochleistungs-Flüssigchromatographie (HI-HPLC) an der Zusammensetzung ausgeführt wird, die Zusammensetzung eine Peak 3-Menge von etwa 12 Gew.-% oder weniger enthält.

2. Zusammensetzung nach Anspruch 1, wobei der Elutionspuffer (a) etwa 250-400 mM Ammoniumsulfat oder Natriumsulfat oder etwa 450-750 mM NaCl oder Ammoniumchlorid und (b) etwa 10-200 mM Natriumacetat, Natriumcitrat oder -phosphat umfasst.

3. Zusammensetzung nach Anspruch 1 oder 2, wobei der Elutionspuffer einen pH-Wert von 3-7 aufweist.

4. Zusammensetzung nach Anspruch 1-3, wobei das Verfahren ferner den Schritt (b1) vor Schritt (c) umfasst: (b1) Waschen von Verunreinigungen von dem HIC-Harz mit einem Waschpuffer.

5. Zusammensetzung nach Anspruch 4, wobei der Waschpuffer (a) etwa 500-800 mM Ammoniumsulfat oder Natriumsulfat oder etwa 1000-1500 mM NaCl oder Ammoniumchlorid und (b) etwa 10-200 mM Natriumacetat, Natriumcitrat oder -phosphat umfasst.

6. Zusammensetzung nach Anspruch 4 oder 5, wobei der Waschpuffer einen pH-Wert von 3-7 aufweist.

7. Zusammensetzung nach Anspruch 4-6, wobei die Leitfähigkeit des Waschpuffers höher ist als die Leitfähigkeit des Elutionspuffers oder die Molarität des Elutionspuffers niedriger ist als die Molarität des Waschpuffers.

8. Zusammensetzung nach einem der Ansprüche 1-7, wobei das reduzierte Niveau von Immunogenität ein reduziertes Niveau von Anti-Arzneimittelantikörpern (ADA) ist, das 50 %, 60 %, 70 %, 80 %, 90 % oder mehr mit Bezug auf ein Subjekt, dem eine ENBREL^{WZ}-Etanercept-Zusammensetzung mit einer äquivalenten Konzentration von Etanercept verabreicht worden ist, reduziert ist.

9. Zusammensetzung nach einem der Ansprüche 1-8, wobei die Zusammensetzung in einer Fertigspritze verpacket ist.

10. Zusammensetzung nach einem der Ansprüche 1-9, wobei die Zusammensetzung für das subkutane Einspritzen geeignet ist.

11. Zusammensetzung nach einem der Ansprüche 1-10, wobei die Mischung aus Schritt (a) 8,0 ng/l oder weniger Wirtszellproteine (HCP) enthält.

12. Zusammensetzung nach einem der Ansprüche 1-11, wobei, wenn hydrophobe Wechselwirkungshochleistungs-Flüssigchromatographie (HI-HPLC) an der Zusammensetzung ausgeführt wird, die Zusammensetzung eine Peak 3-Menge von etwa 7 Gew.-% oder weniger enthält.

13. Zusammensetzung nach Anspruch 12, wobei die Zusammensetzung eine Peak 3-Menge in einem Bereich von etwa 3,5-7 Gew.-% enthält.

14. Zusammensetzung nach Anspruch 12 oder 13, wobei die Peak 3-Menge 50 %, 60 %, 70 % oder mehr mit Bezug auf die Peak 3-Menge, die in der ENBREL^{WZ}-Etanercept-Zusammensetzung mit einer äquivalenten Konzentration von Etanercept enthalten ist und HPLC unter denselben Bedingungen unterworfen worden ist, reduziert ist.

15. Zusammensetzung nach einem der Ansprüche 1-14, wobei das Verfahren zusätzlich eines oder mehrere der Folgenden umfasst: Ionenaustauschchromatographie, Affinitätschromatographie, Größenausschlusschromatographie und Virusinaktivierung.

16. Zusammensetzung nach einem der Ansprüche 1-15, umfassend 10 mg/ml bis 100 mg/ml Etanercept oder 50 mg/ml Etanercept.

17. Zusammensetzung nach einem der Ansprüche 1-16, wobei die Konzentration von fehlgefalteten und angesammelten Etanerceptformen in der Zusammensetzung 50 %, 60 %, 70 %, 80 %, 90 % oder mehr mit Bezug auf die Konzentration von fehlgefalteten und angesammelten Etanerceptformen in der ENBREL^{WZ}-Etanercept-Zusammensetzung reduziert ist.

18. Zusammensetzung nach einem der Ansprüche 1-17, wobei die Konzentration von hochmolekularen Verbindungen in der Zusammensetzung 50 %, 60 %, 70 %, 80 %, 90 % oder mehr mit Bezug auf die Konzentration von hochmolekularen Verbindungen in der ENBREL^{WZ}-Etanercept-Zusammensetzung reduziert ist.

19. Zusammensetzung nach einem der Ansprüche 1-18 zur Verwendung bei der Behandlung eines Leidens ausgewählt aus der Gruppe bestehend aus polyartikulärer juveniler idiopathischer Arthritis (JIA), psoriatischer Arthritis, Psoriasis vulgaris und ankylosierender Spondylitis oder zur Verwendung bei der Behandlung rheumatischer Arthritis.

20. Zusammensetzung zur Verwendung nach Anspruch 19, wobei die Zusammensetzung subkutan verabreicht wird.

21. Zusammensetzung zu Verwendung nach Anspruch 19, wobei in dem Subjekt das Niveau von ADA 50 %, 60 %, 70 %, 80 %, 90 % oder mehr mit Bezug auf ein Subjekt, dem eine ENBREL^{WZ}-Etanercept-Zusammensetzung mit einer äquivalenten Konzentration von Etanercepts verabreicht worden ist, reduziert ist.

22. Verfahren zum Erhalten einer Zusammensetzung umfassend ein Anti-Tumor-Nekrose-Faktor- (TNF) alpha-Polypeptid, wobei das Anti-TNF-alpha-Polypeptid Etanercept ist, umfassend:
(a) Bereitstellen einer Mischung umfassend Etanercept und Verunreinigungen umfassend Anhäufungen und fehlgefaltetes Etanercept,
(b) Kontaktieren der Mischung mit einem hydrophoben Wechselwirkungschromatographie- (HIC) Harz, derart, dass das Etanercept sich an das HIC-Harz bindet, und
(c) Eluieren des Etanercepts aus dem HIC-Harz mit einem Elutionspuffer,
(d) Erhalten der Zusammensetzung umfassend Etanercept,
wobei die Zusammensetzung ein reduziertes Niveau von Immunogenität, wenn es einem Subjekt verabreicht wird, im Vergleich mit einer ENBREL^{WZ}-Etanercept-Zusammensetzung mit einer äquivalenten Konzentration von Etanercept erzeugt.

## Revendications

1. Composition comprenant un polypeptide anti-facteur de nécrose tumorale (TNF)-alpha, dans laquelle le polypeptide anti-TNF-alpha est l'étanercept,
dans laquelle la composition est préparée par un procédé comprenant :
(a) la fourniture d'un mélange comprenant de l'étanercept et des impuretés comprenant des agrégats d'étanercept et des formes mal repliées d'étanercept,
(b) la mise en contact du mélange avec une résine pour chromatographie d'interaction hydrophobe (HIC), de telle sorte que l'étanercept se lie à la résine HIC, et
(c) l'élution de l'étanercept de la résine HIC avec un tampon d'élution, obtenant ainsi la composition comprenant de l'étanercept
dans laquelle ladite composition produit un taux réduit d'immunogénicité chez le sujet par comparaison à une composition d'étanercept ENBREL^{™} avec une concentration équivalente d'étanercept, et
dans laquelle, lorsqu'une chromatographie liquide haute performance d'interaction hydrophobe (HI-HPLC) est réalisée sur la composition, la composition contient une quantité de Pic 3 d'environ 12 % en poids ou moins.

2. Composition selon la revendication 1 dans laquelle le tampon d'élution comprend (a) d'environ 250 à 400 mM de sulfate d'ammonium ou de sulfate de sodium ou d'environ 450 à 750 mM de NaCl ou de chlorure d'ammonium et (b) d'environ 10 à 200 mM d'acétate de sodium, de citrate ou de phosphate de sodium.

3. Composition selon la revendication 1 ou 2 dans laquelle le tampon d'élution a un pH de 3 à 7.

4. Composition selon l'une quelconque des revendications 1 à 3, dans laquelle le procédé comprend en outre l'étape (b1) avant l'étape (c) : (b1) le lavage des impuretés de la résine HIC avec un tampon de lavage.

5. Composition selon la revendication 4, dans laquelle le tampon de lavage comprend (a) d'environ 500 à 800 mM de sulfate d'ammonium ou de sulfate de sodium ou d'environ 1000 à 1500 mM de NaCl ou de chlorure d'ammonium et (b) d'environ 10 à 200 mM d'acétate de sodium, de citrate ou de phosphate de sodium.

6. Composition selon la revendication 4 ou 5, dans laquelle le tampon de lavage a un pH de 3 à 7.

7. Composition selon l'une quelconque des revendications 4 à 6, dans laquelle la conductivité du tampon de lavage est supérieure à la conductivité du tampon d'élution, ou la molarité du tampon d'élution est inférieure à la molarité du tampon de lavage.

8. Composition selon l'une quelconque des revendications 1 à 7, dans laquelle le taux réduit d'immunogénicité est un taux réduit d'anticorps anti-médicament (ADA) qui est réduit de 50 %, 60 %, 70 %, 80 %, 90 % ou plus par rapport à un sujet auquel est administrée une composition d'étanercept ENBREL^{™} avec une concentration équivalente d'étanercept.

9. Composition selon l'une quelconque des revendications 1 à 8, dans laquelle la composition est conditionnée dans une seringue préremplie.

10. Composition selon l'une quelconque des revendications 1 à 9, dans laquelle la composition est appropriée pour une injection sous-cutanée.

11. Composition selon l'une quelconque des revendications 1 à 10, dans laquelle le mélange de l'étape (a) contient 8,0 ng/L ou moins de protéines de cellules hôtes (HCP).

12. Composition selon l'une quelconque des revendications 1 à 11, dans laquelle, lorsqu'une chromatographie liquide haute performance d'interaction hydrophobe (HI-HPLC) est réalisée sur la composition, la composition contient une quantité de Pic 3 d'environ 7 % en poids ou moins.

13. Composition selon la revendication 12, dans laquelle la composition contient une quantité de Pic 3 dans une plage d'environ 3,5 à 7 % en poids.

14. Composition selon la revendication 12 ou 13, dans laquelle la quantité de Pic 3 est réduite de 50 %, 60 %, 70 % ou plus par rapport à la quantité de Pic 3 contenue dans la composition d'étanercept ENBREL^{™} avec une concentration équivalente d'étanercept et soumise à une HPLC dans les mêmes conditions.

15. Composition selon l'une quelconque des revendications 1 à 14, dans laquelle le procédé comprend en outre une ou plusieurs de ce qui suit : une chromatographie par échange d'ions, une chromatographie d'affinité, une chromatographie d'exclusion de taille et une inactivation du virus.

16. Composition selon l'une quelconque des revendications 1 à 15, comprenant de 10 mg/mL à 100 mg/mL d'étanercept ou 50 mg/mL d'étanercept.

17. Composition selon l'une quelconque des revendications 1 à 16, dans laquelle la concentration de formes mal repliées et agrégées d'étanercept dans la composition est réduite de 50 %, 60 %, 70 %, 80 %, 90 % ou plus par rapport à la concentration de formes mal repliées et agrégées d'étanercept dans la composition d'étanercept ENBREL^{™}.

18. Composition selon l'une quelconque des revendications 1 à 17 dans laquelle la concentration des composés de poids moléculaire élevé dans la composition est réduite de 50 %, 60 %, 70 %, 80 %, 90 % ou plus par rapport à la concentration des composés de poids moléculaire élevé dans la composition d'étanercept ENBREL^{™}.

19. Composition selon l'une quelconque des revendications 1 à 18 pour une utilisation dans le traitement d'un trouble choisi dans le groupe constitué par l'arthrite juvénile idiopathique (AJI) polyarticulaire, l'arthrite psoriasique, le psoriasis en plaques et la spondylarthrite ankylosante, ou pour une utilisation dans le traitement de la polyarthrite rhumatoïde.

20. Composition pour une utilisation selon la revendication 19, dans laquelle la composition est administrée par voie sous-cutanée.

21. Composition pour une utilisation selon la revendication 19, dans laquelle chez le sujet le taux d'ADA est réduit de 50 %, 60 %, 70 %, 80 %, 90 % ou plus par rapport à un sujet auquel est administrée une composition d'étanercept ENBREL^{™} avec une concentration équivalente d'étanercept.

22. Procédé d'obtention d'une composition comprenant un polypeptide anti-facteur de nécrose tumorale (TNF)-alpha dans lequel le polypeptide anti-TNF-alpha est l'étanercept, comprenant :
(a) la fourniture d'un mélange comprenant de l'étanercept et des impuretés comprenant des agrégats d'étanercept et de l'étanercept mal replié,
(b) la mise en contact du mélange avec une résine pour chromatographie d'interaction hydrophobe (HIC), de telle sorte que l'étanercept se lie à la résine HIC, et
(c) l'élution de l'étanercept de la résine HIC avec un tampon d'élution,
(d) l'obtention de la composition comprenant de l'étanercept
dans lequel ladite composition produit un taux réduit d'immunogénicité lorsqu'elle est administrée au sujet par comparaison à une composition d'étanercept ENBREL^{™} avec une concentration équivalente d'étanercept.
